# EUROPEAN PATENT APPLICATION

(11) **EP 4 159 757 A1**
(43) Date of publication of application: **05.04.2023**
(21) Application number: 21817035.5
(22) Date of filing: 08.03.2021
(51) Int. Cl.: C07K 16/10, C12N 15/13, C12N 15/63, C12P 21/08, A61K 39/395, A61K 47/68, A61P 31/14, G01N 33/569

(54) **SARS-COV-2 SPIKE PROTEIN BINDING MOLECULE AND APPLICATION THEREOF**

(30) Priority: 02.06.2020 CN 202010491549
(71) Applicant: Shenzhen Immunotherapy Biotech Co., Ltd., Shenzhen, Guangdong 518000 (CN)
(72) Inventor: ZHANG, Junfang, Shenzhen, Guangdong 518000 (CN)
(74) Representative: Delbarba, Andrea
(86) International application number: PCT/CN2021/079568
(87) International publication number: WO 2021/244089

(57) **Abstract**

Provided are a SARS-COV-2 spike protein binding molecule and an application thereof. The binding molecule may specifically bind to a SARS-COV-2 spike protein and contains at least one immunoglobulin single variable domain, may effectively block the binding of the SARS-COV-2-Spike protein to an ACE2 receptor of a human cell, thereby blocking the infection of the cell with SARS-COV-2 and inhibiting SARS-COV-2 infection and amplification.

## Description

### Cross-Reference to Related Application

The present application claims priority to Chinese patent application CN202010491549.0, filed on June 2, 2020, the disclosure of which is hereby incorporated by reference in its entirety.

### Technical Field

The present application relates to a novel coronavirus (SARS-COV-2) spike protein binding molecule and an application thereof, and belongs to the field of medical biotechnologies.

### Background

Novel coronavirus pneumonia (COVID-19) infects more than 4 million people worldwide cumulatively, and the number of infected persons is still increased rapidly. At present, there is no specific and effective clinical treatment means for COVID-19. Although the epidemic situation in China is fully controlled already, the epidemic situation abroad breaks out, and is still grown rapidly. In addition, more and more researches show that the SARS-COV-2 infection may have a chronic carrier state; and some discharged patients with positive symptoms also suggest that the virus may exist in a human body for a long time. At present, it is not clear about key factors such as mechanism and time of the long-term carrier existence, and it is very important to prevent the resurgence of SARS-COV-2 in the future. Affected by COVID-19, the economic losses, social burdens and other negative impacts caused by this in China and other countries in the world are difficult to measure.

At present, there is no specific drug for COVID-19, and there is an urgent need to develop an effective drug quickly. Many research and development institutions at home and abroad race against the clock on the research of treatment strategies for COVID-19. Although broad-spectrum small-molecule antiviral drugs such as remdesivir and favipiravir that are already discovered have a certain curative effect on COVID-19, due to the lack of specificity for SARS-COV-2, the therapeutic effect is limited, and it is difficult to become a specific drug for COVID-19.

### Summary

### Technical problem

In view of the problems that the existing antiviral drugs are not specific to SARS-COV-2, the therapeutic effect is limited, and it is difficult to become the specific drug for SARS-COV-2, the present application provides a SARS-COV-2 spike protein binding molecule and an application thereof.

### Technical application

A first aspect of the present application provides a SARS-COV-2 spike protein binding molecule that may specifically bind to a SARS-COV-2 spike protein and contains at least one immunoglobulin single variable domain, and CDR1, CDR2 and CDR3 in the immunoglobulin single variable domain are selected from any one of the following combinations:
1) CDR1 shown in SEQ ID NO: 1, CDR2 shown in SEQ ID NO: 2 and CDR3 shown in SEQ ID NO: 3;
2) CDR1 shown in SEQ ID NO: 4, CDR2 shown in SEQ ID NO: 5 and CDR3 shown in SEQ ID NO: 6;
3) CDR1 shown in SEQ ID NO: 7, CDR2 shown in SEQ ID NO: 8 and CDR3 shown in SEQ ID NO: 9;
4) CDR1 shown in SEQ ID NO: 10, CDR2 shown in SEQ ID NO: 11 and CDR3 shown in SEQ ID NO: 12;
5) CDR1 shown in SEQ ID NO: 13, CDR2 shown in SEQ ID NO: 14 and CDR3 shown in SEQ ID NO: 15;
6) CDR1 shown in SEQ ID NO: 16, CDR2 shown in SEQ ID NO: 17 and CDR3 shown in SEQ ID NO: 18;
7) CDR1 shown in SEQ ID NO: 19, CDR2 shown in SEQ ID NO: 20 and CDR3 shown in SEQ ID NO: 21;
8) CDR1 shown in SEQ ID NO: 22, CDR2 shown in SEQ ID NO: 23 and CDR3 shown in SEQ ID NO: 24;
9) CDR1 shown in SEQ ID NO: 25, CDR2 shown in SEQ ID NO: 26 and CDR3 shown in SEQ ID NO: 27;
10) CDR1 shown in SEQ ID NO: 28, CDR2 shown in SEQ ID NO: 29 and CDR3 shown in SEQ ID NO: 30;
11) CDR1 shown in SEQ ID NO: 31, CDR2 shown in SEQ ID NO: 32 and CDR3 shown in SEQ ID NO: 33;
12) CDR1 shown in SEQ ID NO: 34, CDR2 shown in SEQ ID NO: 35 and CDR3 shown in SEQ ID NO: 36;
13) CDR1 shown in SEQ ID NO: 37, CDR2 shown in SEQ ID NO: 38 and CDR3 shown in SEQ ID NO: 39;
14) CDR1 shown in SEQ ID NO: 40, CDR2 shown in SEQ ID NO: 41 and CDR3 shown in SEQ ID NO: 42;
15) CDR1 shown in SEQ ID NO: 43, CDR2 shown in SEQ ID NO: 44 and CDR3 shown in SEQ ID NO: 45;
16) CDR1 shown in SEQ ID NO: 46, CDR2 shown in SEQ ID NO: 47 and CDR3 shown in SEQ ID NO: 48;
17) CDR1 shown in SEQ ID NO: 49, CDR2 shown in SEQ ID NO: 50 and CDR3 shown in SEQ ID NO: 51;
18) CDR1 shown in SEQ ID NO: 52, CDR2 shown in SEQ ID NO: 53 and CDR3 shown in SEQ ID NO: 54;
19) CDR1 shown in SEQ ID NO: 55, CDR2 shown in SEQ ID NO: 56 and CDR3 shown in SEQ ID NO: 57;
20) CDR1 shown in SEQ ID NO: 58, CDR2 shown in SEQ ID NO: 59 and CDR3 shown in SEQ ID NO: 60;
21) CDR1 shown in SEQ ID NO: 61, CDR2 shown in SEQ ID NO: 62 and CDR3 shown in SEQ ID NO: 63;
22) CDR1 shown in SEQ ID NO: 64, CDR2 shown in SEQ ID NO: 65 and CDR3 shown in SEQ ID NO: 66;
23) CDR1 shown in SEQ ID NO: 67, CDR2 shown in SEQ ID NO: 68 and CDR3 shown in SEQ ID NO: 69;
24) CDR1 shown in SEQ ID NO: 70, CDR2 shown in SEQ ID NO: 71 and CDR3 shown in SEQ ID NO: 72;
25) CDR1 shown in SEQ ID NO: 73, CDR2 shown in SEQ ID NO: 74 and CDR3 shown in SEQ ID NO: 75;
26) CDR1 shown in SEQ ID NO: 76, CDR2 shown in SEQ ID NO: 77 and CDR3 shown in SEQ ID NO: 78; and
27) CDR1 shown in SEQ ID NO: 79, CDR2 shown in SEQ ID NO: 80 and CDR3 shown in SEQ ID NO: 81.

As an embodiment of the present application, the immunoglobulin single variable domain is a single domain antibody.

As an embodiment of the present application, the single domain antibody contains an amino acid sequence having at least 80% of the sequence identity with any one of SEQ ID NOs: 82-108.

As an embodiment of the present application, the single domain antibody contains an amino acid sequence having at least 90% of the sequence identity with any one of SEQ ID NOs: 82-108.

As an embodiment of the present application, the single domain antibody contains an amino acid sequence having at least 99% of the sequence identity with any one of SEQ ID NOs: 82-108.

As an embodiment of the present application, the amino acid sequence of the single domain antibody contains one or more amino acid substitutions, preferably a conservative amino acid substitution, compared with any one of SEQ ID NOs: 82-108.

As an embodiment of the present application, the single domain antibody contains any one of the amino acid sequences of SEQ ID NOs: 82-108.

As an embodiment of the present application, the SARS-COV-2 spike protein binding molecule further contains an immunoglobulin Fc region.

The inclusion of the immunoglobulin Fc region in the SARS-COV-2 spike protein binding molecule of the present application allows the binding molecule to form a dimer while the in vivo half-life of the molecule is further prolonged. The Fc region suitable for the present application may be derived from different subtypes of immunoglobulins, for example, IgG (IgGI, IgG2, IgG3 or IgG4 subtype), IgAI, IgA2, IgD, IgE or IgM.

As an embodiment of the present application, the immunoglobulin Fc region is a human immunoglobulin Fc region.

As an embodiment of the present application, the immunoglobulin Fc region is an Fc region of human lgG1.

As an embodiment of the present application, an amino acid sequence of the immunoglobulin Fc region is SEQ ID NO: 109.

As an embodiment of the present application, at least one amino acid sequence of SEQ ID NOs: 110-136 is contained.

The stability and biological activity of the binding molecule fused with the above Fc region are further improved, and the dissociation equilibrium constant (KD) value of its binding to the SARS-COV-2 spike protein is further reduced.

As an embodiment of the present application, the SARS-COV-2 spike protein binding molecule has at least one of the following characteristics:
a. the KD value of binding to the SARS-COV-2 spike protein is less than 1×10⁻⁸ M;
b. the binding of SARS-COV-2 to a human cell receptor ACE2 is blocked; and
c. the infection and amplification of SARS-COV-2 are inhibited.

A second aspect of the present application provides a nucleic acid molecule encoding the SARS-COV-2 spike protein binding molecule, the nucleic acid molecule is a ribonucleic acid (RNA), a deoxyribonucleic acid (DNA) or a complementary deoxyribonucleic acid (cDNA), and it may be obtained by artificial synthesis, or may be obtained by isolated from a suitable natural source.

A third aspect of the present application provides an expression vector containing the nucleic acid molecule and its expression regulatory element. The expression vector usually contains at least one nucleic acid molecule provided in the present application, and it is operably linked to one or more suitable expression regulatory elements (a promoter, an enhancer, a terminator, an integration factor, a selectable marker, a leader sequence, a reporter gene and the like). The selection of the element and the sequence thereof for expression in a specific host cell is within the general knowledge of those skilled in the art.

A fourth aspect of the present application provides a host cell containing and expressing the nucleic acid molecule. The host cell is a cell used for expressing a heterologous protein, including a bacterial cell, a fungal cell or a mammalian cell.

A fifth aspect of the present application provides a method for acquiring the SARS-COV-2 spike protein binding molecule, including:
a, the above host cell is cultured under a condition that the expression of the SARS-COV-2 spike protein binding molecule is allowed; and
b. the SARS-COV-2 spike protein binding molecule expressed by the host cell is collected from a culture in step a.

Recombination of a specific nucleic acid molecule into the expression vector and expression in the host cell by transformation or transfection methods, selection of makers, methods of inducing protein expression, culture conditions and the like are known in the art. At the same time, separation and purification technologies of the protein binding molecule are well-known to those skilled in the art.

The SARS-COV-2 spike protein binding molecule provided in the present application may also be obtained by other methods known in the art for producing proteins, such as chemical synthesis.

A sixth aspect of the present application provides an immunoconjugate, containing the SARS-COV-2 spike protein binding molecule of any one of the above conjugated to a therapeutic moiety.

A seventh aspect of the present application provides a pharmaceutical composition, containing the SARS-COV-2 spike protein binding molecule and/or the immunoconjugate of any one of the above, and a pharmaceutically acceptable carrier.

The "pharmaceutically acceptable carrier" in the present application includes any solvents, dispersion media, coatings, antibacterial and antifungal agents, isotonic agents and absorption delaying agents and the like that are physiologically compatible. The carrier is suitable for intravenous, intramuscular, subcutaneous, parenteral, spinal or epidermal administration (for example, by injection or infusion). According to the route of administration, an active compound, namely the binding molecule or the immunoconjugate, may be encapsulated in a material, as to protect the compound from the action of an acid and other natural conditions that may inactivate the compound, and it is well-known to those skilled in the art.

The pharmaceutical composition in the present application may also include other adjuvants and excipients as needed.

An eighth aspect of the present application provides use of the pharmaceutical composition in preparation of a drug for treating or preventing COVID-19.

A ninth aspect of the present application provides a kit for detecting SARS-COV-2, containing the SARS-COV-2 spike protein binding molecule of any one of the above.

A tenth aspect of the present application provides a using method of the kit for detecting SARS-COV-2, under a condition that a complex may be formed between the SARS-COV-2 spike protein binding molecule of any one of the above and the SARS-COV-2 spike protein, a detection sample and a control sample are contacted with the SARS-COV-2 spike protein binding molecule of any one of the above, and the formation of the complex is detected; and the presence of SARS-COV-2 in the sample is judged by a difference in complex formation between the detection sample and the control sample.

### Beneficial effect

The SARS-COV-2-Spike protein binding molecule provided in the present application may specifically bind to the SARS-COV-2-Spike protein, and effectively block the binding of the SARS-COV-2-Spike protein to the ACE2 receptor of the human cell, thereby the infection process of SARS-COV-2 on the cell is blocked, and the infection and amplification of SARS-COV-2 are inhibited. In addition, the SARS-COV-2-Spike protein binding molecule provided in the present application also has the characteristics of good binding specificity with the SARS-COV-2-Spike protein, high biological activity and stability, and no toxic and side effects.

### Brief Description of the Drawings

Fig. 1 is an agarose gel electrophoresis image of total RNA extracted in Embodiment 1 of the present application, herein M: DNA marker 2000, and lane 1: total RNA.
Fig. 2 is an agarose gel electrophoresis image of a Step1-PCR amplification product of a single domain antibody gene amplified by a nested polymerase chain reaction (PCR) in Embodiment 1 of the present application, herein M: DNA marker 2000, and lane 1: amplification product.
Fig. 3 is an agarose gel electrophoresis image of a Step2-PCR amplification product of the single domain antibody gene amplified by the nested PCR in Embodiment 1 of the present application, herein, DNA marker 2000, lanes 1 and 2: amplification products.
Fig. 4 is an agarose gel electrophoresis image of Sfi I and Not I double enzyme-digested products of a target single domain antibody gene and a vector pHEN1 in Embodiment 1 of the present application, herein DNA marker 2000, lane 1: pHEN1; lane 2: pHEN1 after Sfi I/Not I enzyme digestion; and lane 3: single domain antibody gene after Sfi I/Not I enzyme digestion.
Fig. 5 is an agarose gel electrophoresis image of a colony PCR amplification product for measuring a library insertion rate in Embodiment 1 of the present application, herein M: DNA marker 2000; lanes 1-48: 48 picked colonies.
Fig. 6 is a diagram showing a change of viral load of rhesus monkeys in a treatment group and a control group with a change of days in Embodiment 2 of the present application.

### Detailed Description of the Embodiments

### Definition

Unless otherwise indicated or defined, all terms used have ordinary meaning in the art, and this meaning is understood by those skilled in the art. Furthermore, unless otherwise stated, all methods, steps, technologies, and operations which are not specifically detailed may be already performed in a mode known by itself, and the mode is understood by those skilled in the art.

Unless otherwise stated, a term "antibody" or "immunoglobulin" which is used interchangeably herein, whether referring to a heavy-chain antibody or a conventional 4-chain antibody, is used as a general term to include a full-length antibody, its single chain and all moieties thereof, domains or fragments (including but not limited to an antigen binding domain or fragment). Furthermore, a term "sequence" used herein (for example, in terms of "immunoglobulin sequence", "antibody sequence", "single variable domain sequence", "single domain antibody sequence" or "protein sequence" and the like) should be generally understood to include both a related amino acid sequence and a nucleic acid sequence or a nucleotide sequence encoding the sequence, unless the more limited explanation is required herein.

A term "immunoglobulin variable domain" as used herein refers to an immunoglobulin domain composed of four "framework regions" which are essentially defined in the art and hereinafter referred to as "framework region 1" or "FR1", "framework region 2" or "FR2", "framework region 3" or "FR3", and "framework region 4" or "FR4", herein the framework regions are spaced by three "complementarity determining regions" or "CDRs" which are defined in the art and hereinafter referred to as "complementarity determining region 1" or "CDR1", "complementarity determining region 2" or "CDR2", and "complementarity determining region 3" or "CDR3". Therefore, the general structure or sequence of the immunoglobulin variable domain may be represented as follows: FR1-CDR1-FR2-CDR2-FR3-CDR3-FR4. The immunoglobulin variable domain confers the antibody specificity to the antigen because it has an antigen-binding site.

A traditional IgG antibody molecule is generally composed of a light chain and a heavy chain, the light chain contains 1 variable region (VL) and 1 constant region (CL), and the heavy chain contains 1 variable region (VH) and 3 constant regions (CH1, CH2, CH3). The single domain antibody (sdAb) refers to a type of the antibody that lacks the light chain of the antibody and only has the variable region of the heavy chain. Because of its small molecular weight, it is also called a nanobody. The single domain antibody specifically binds to an epitope without the need for other antigen-binding domains. The single domain antibody is a small, stable and high-efficient antigen-recognition unit formed by the single immunoglobulin domain.

As described in the field, the total number of amino acid residues in each CDR in the single domain antibody may be different.

The total number of the amino acid residues in the single domain antibody may typically range from 110 to 120, often between 112 and 115. However, it should be noted that smaller and longer sequences may also be suitable for the purpose of the present application.

Other structural and functional properties of the single domain antibody and a polypeptide containing thereof may be summarized as follows.

The single domain antibody (it is naturally "designed" already to functionally bind to the antigen in the case of the absence of the light chain variable domain and without the interaction with the light chain variable domain) may serve as a single and relatively small functional antigen-binding structural unit, domain or polypeptide. This distinguishes the single domain antibody from the VH and VL domains of the conventional 4-chain antibody, these VH and VL domains are generally not suitable for practical applications as the single antigen binding protein or the immunoglobulin single variable domain by themselves, but a certain form or another form is required to be combined to provide a functional antigen binding unit (for example, in the form of a conventional antibody fragment such as an Fab fragment; or in the form of scFvs consisting of the VH domain covalently linked to the VL domain).

Because of these unique properties described above, the use of the single domain antibody or as a part of a larger polypeptide provides many significant advantages over the use of the conventional VH and VL domains, scFvs, or conventional antibody fragments (for example, Fab- or F(ab')2-fragment), for example, the single domain antibody requires only the single domain to bind to the antigen with high affinity and high selectivity, so that neither the presence of two separate domains nor the need to ensure that the two domains present in the proper spatial conformation and configuration (for example, scFvs typically require the use of a specially designed linker) are required; the single domain antibody may be expressed from a single gene and does not require post-translational folding or modification; the single domain antibody may be easily engineered into a multivalent and multispecific format; the single domain antibody is highly soluble and has no tendency to aggregate; the single domain antibody is highly stable to heat, pH, a protease and other denaturing agents or conditions, and therefore may be prepared, stored or transported without the use of a refrigeration device, thereby the cost, time and environment are saved; the single domain antibody is easy and relatively inexpensive to prepare, even in the scale required for production; the single domain antibody is relatively small (approximately 15 kDa or 1/10 of the size of the conventional IgG) compared to the conventional 4-chain antibody and its antigen-binding fragment, and thus shows the higher tissue penetration and may be administered at the higher dose compared to the conventional 4-chain antibody and its antigen-binding fragment; and the single domain antibody may show so-called cavity-binding properties (especially due to its extended CDR3 loop compared with the conventional VH domain), thereby it may reach a target and an epitope that are inaccessible to the conventional 4-chain antibody and antigen-binding fragments thereof.

Methods for acquiring the single domain antibody that binds to the specific antigen or epitope are previously disclosed already in the following documents: R. van der Linden et al., Journal of Immunological Methods, 240 (2000) 185-195; Li et al., JBiol Chem., 287(2012) 13713-13721; Deffar et al., African Journal of Biotechnology Vol. 8(12), pp. 2645-2652, 17 June, 2009 and WO94/04678.

In addition, those skilled in the art may also understand that it is possible to "graft" one or more of the above CDRs onto other "scaffolds" (including, but not limited to, a human scaffold or a non-immunoglobulin scaffold). The scaffolds and technologies suitable for the CDR grafting are known in the field.

In general, a term "specificity" refers to the number of different types of the antigens or epitopes to which a specific antigen-binding molecule or an antigen-binding protein (for example, the immunoglobulin single variable domain of the present application) molecule may bind. Its specificity may be determined on the basis of the affinity and/or avidity of the antigen-binding molecule. The affinity, represented by KD of the antigen and the antigen-binding protein, is a measure of the binding strength between the epitope and the antigen-binding site on the antigen-binding protein: the KD value is smaller, and the affinity between the epitope and the antigen-binding molecule is greater (alternatively, the affinity may also be represented as an association constant (KA), and it is 1/KD). For example, those skilled in the art may understand that the affinity may be measured in a known manner depending on the specific antigen of interest.

The avidity is the measure of the binding strength between the antigen-binding molecule (for example, the immunoglobulin, the antibody, the immunoglobulin single variable domain or the polypeptide containing it) and the related antigens. The avidity is related to the following both: the affinity between the antigen-binding sites on the antigen-binding molecule thereof, and the number of the related binding sites present on the antigen-binding molecule.

A term "SARS-COV-2 spike protein binding molecule (SARS-COV-2-Spike protein binding molecule)" as used in the present application means any molecules capable of specifically binding to the SARS-COV-2 spike protein. The SARS-COV-2 spike protein binding molecule may include a single domain antibody as defined in the present application, or a conjugate thereof, directed against the SARS-COV-2 spike protein. The SARS-COV-2 spike protein binding molecule also encompasses a so-called "small modular immunopharmaceutical (SMIP)", or an immunoglobulin superfamily antibody (IgSF) or a CDR grafting molecule.

The "SARS-COV-2 spike protein binding molecule" of the present application may include at least one immunoglobulin single variable domain such as the single domain antibody that binds to the SARS-COV-2 spike protein. In some implementation embodiments, the "SARS-COV-2 spike protein binding molecule" of the present application may include two immunoglobulin single variable domains that bind to the SARS-COV-2 spike protein, such as the single domain antibody. The SARS-COV-2 spike binding molecule containing more than one immunoglobulin single variable domain are also referred to as a "formatted" SARS-COV-2 spike binding molecule. The formatted SARS-COV-2 spike protein binding molecule may also contain a linker and/or a moiety with an effector function, such as a half-life extending moiety (for example, the immunoglobulin single variable domain that binds to serum albumin), and/or a fusion partner (for example, the serum albumin) and/or a conjugated polymer (for example, polyethylene glycol (PEG)) and/or an Fc region, in addition to the immunoglobulin single variable domain binding to the SARS-COV-2 spike protein. The "SARS-COV-2 spike protein binding molecule" of the present application also encompasses a bispecific antibody, and it contains the immunoglobulin single variable domain that binds to the different antigens.

Typically, the SARS-COV-2 spike protein binding molecule of the present application may be present at preferably 10⁻⁸ to 10⁻¹² mole/liter (M), more preferably 10⁻⁹ to 10⁻¹¹ M, even more preferably 10⁻¹⁰ to 10⁻¹² M, even more preferably 10⁻¹¹ to 10⁻¹² M or the lower KD as measured in a Biacore or KinExA assay. Any KD values greater than 10⁻⁴ M are generally considered to be indicative of nonspecific binding. The specific binding of the antigen-binding protein to the antigen or the epitope may be measured in any suitable manner known, including, for example, a surface plasmon resonance (SPR) assay described herein, and/or a competitive binding assay (for example, an enzyme immunoassay (EIA) and a sandwich competitive assay).

The amino acid residue may be represented according to a standard three-letter or one-letter amino acid code as well-known and agreed in the art. The conservative amino acid substitution is well-known in the art, for example the conservative amino acid substitution is preferably that one amino acid residue within the following groups (1)-(5) is substituted by another amino acid residue within the same group: (1) smaller aliphatic non-polar or weak-polar residue: Ala, Ser, Thr, Pro and Gly; (2) polar negatively charged residue and its (uncharged) amide: Asp, Asn, Glu and Gln; (3) polar positively charged residue: His, Arg and Lys; (4) larger aliphatic non-polar residue: Met, Leu, Ile, Val and Cys; and (5) aromatic residue: Phe, Tyr and Trp. The particularly preferred conservative amino acid substitutions are as follows: Ala is substituted by Gly or Ser; Arg is substituted by Lys; Asn is substituted by Gin or His; Asp is substituted by Glu; Cys is substituted by Ser; Gln is substituted by Asn; Glu is substituted by Asp; Gly is substituted by Ala or Pro; His is substituted by Asn or Gln; lie is substituted by Leu or Val; Leu is substituted by lie or Val; Lys is substituted by Arg, Gin or Glu; Met is substituted by Leu, Tyr or Ile; Phe is substituted by Met, Leu or Tyr; Ser is substituted by Thr; Thr is substituted by Ser; Trp is substituted by Tyr; Tyr is substituted by Trp or Phe; and Val is substituted by lie or Leu.

"Sequence identity" between two polypeptide sequences indicates the percentage of identical amino acids between the sequences. Methods for assessing the degree of the sequence identity between the amino acids or nucleotides are known to those skilled in the art. For example, the amino acid sequence identity is typically measured with the sequence analysis software. For example, the identity may be determined through a BLAST program of a NCBI database. For the determination of the sequence identity, referring to, for example: Sequence Analysis in Molecular Biology, von Heinje, G., Academic Press, 1987 and Sequence Analysis Primer, Gribskov, M. and Devereux, J., eds., M Stockton Press, New York, 1991.

Compared to its natural biological source and/or a reaction medium or a culture medium from which the polypeptide or nucleic acid molecule is obtained, while it is already separated from at least one other component (for example, another protein/polypeptide, another nucleic acid, another biological component or macromolecule or at least one contaminant, impurity or minor component), generally related to it, in the source or medium (culture medium), the polypeptide or nucleic acid molecule is considered as "substantially separated". In particular, the polypeptide or nucleic acid molecule is considered as "substantially separated" while it is already purified at least 2-fold, in particular at least 10-fold, more particularly at least 100-fold and up to 1000-fold or more. The "substantially separated" polypeptide or nucleic acid molecule is preferably substantially homogeneous as determined by a suitable technology (for example, a suitable chromatographic technology, such as polyacrylamide gel electrophoresis).

### Embodiment 1

### Screening of single domain antibodies against SARS-COV-2-Spike protein

### 1.1 Construction of library

### 1.1.1 Immunization

An alpaca was immunized with SARS-COV-2 Spike-RBD protein, and immunized at the 1st, 2nd, 4th, and 6th weeks, for a total of 4 times, and each immunization dose was 200 ug.

### 1.1.2 Extraction of total RNA

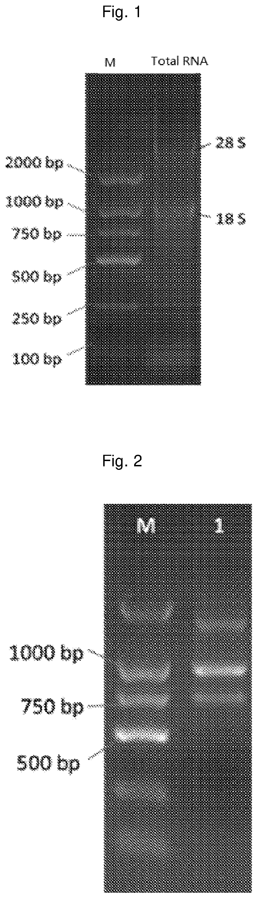

50 ml of peripheral blood of the 6th week immunized alpaca was taken, the lymphocyte was separated, the total RNA of the lymphocyte was extracted with Trizol, and the extracted RNA was detected with an ultraviolet spectrophotometer. Results were: OD260/280=1.99, OD260/230=1.43, it was indicated that the extracted RNA was not significantly degraded, and had the better purity; and the total RNA concentration was 809.3 ng/µL. The extracted total RNA performs agarose gel electrophoresis, and results were shown in Fig. 1. Two bands of 28 S and 18 S may be seen.

### 1.1.3 RNA reverse transcription

The RNA reverse transcription system was as follows.

### Step 1: (Table 1)

**Table 1**

| | |
|---|---|
| Oligo dT (2.5 µM) | 1 µL |
| dNTP (10 mM each) | 1 µL |
| Total RNA | 2.428 µg |
| H₂O | Up to 10 µL |

After mixing uniformly, the temperature was kept at 65°C for 5 min, and an ice bath was performed quickly.

### Step 2: (Table 2)

**Table 2**

| | |
|---|---|
| Step1 reaction solution | 10 µL |
| 5×PrimeScript Buffer | 4 µL |
| RNase inhibitor (40 U/µL) | 0.5 µL |
| PrimeScript RTase (200 U/µL) | 0.5 µL |
| RNase free H₂O | Up to 20 µL |

After mixing uniformly, reverse transcription was performed to obtain cDNA, and the reverse transcription conditions were: 42°C, 30 min; 50°C, 15 min; and 70°C, 15 min.

### 1.1.4 Single domain antibody (VHH) gene amplification

Nested PCR was used to amplify a VHH gene, and a method was as follows:

### Step1: (Table 3)

**Table 3**

| | |
|---|---|
| 10×Ex Taq Buffer (Mg²⁺ plus) | 5 µL |
| dNTP mixture(each 2.5 mM) | 4 µL |
| Primer For-1 (10 mM each) | 1.5 µL |
| Primer Rev-1 (10 mM each) | 1.5 µL |
| cDNA | 1 µL |
| Ex Taq enzyme | 0.25 µL |
| H₂O | Up to 50 µL |

After mixing uniformly, a PCR reaction was performed, and the reaction conditions were: 98°C for 10 s, 50°C for 30 s, 72°C for 1 min, and a total of 20 cycles. The sequences of amplification primers were: Primer For-1: 5'-GTCCTGGCTGCTCTTCTACAAGG-3' (SEQ ID NO: 138); and Primer Rev-1: 5'-GGTACGTGCTGTTGAACTGTTCC-3' (SEQ ID NO: 139).

A PCR product was purified and concentrated by a DNA purification kit, and then subjected to the agarose gel electrophoresis. An obtained agarose gel electrophoresis image was shown in Fig. 2. A DNA product gel recovery kit was used to recover a 750 bp band, and the ultraviolet spectrophotometer was used to quantify;

### Step 2: (Table 4)

**Table 4**

| | |
|---|---|
| 10×Ex Taq Buffer (Mg2+ plus) | 5 µL |
| dNTP mixture(2.5 mM each) | 4 µL |
| Primer For-2 (10 mM each) | 2 µL |
| Primer Rev-2 (10 mM each) | 2 µL |
| Step 1 DNA template | 1 µL |
| Ex Taq | 0.25 µL |
| H₂O | Up to 50 µL |

After mixing uniformly, the PCR reaction was performed, and the reaction conditions were: 98°C for 10 s, 55°C for 30 s, 72°C for 30 s, and a total of 20 cycles. The sequences of the amplification primers were: Primer For-2: 5'- CTAGTGCGGCCGCTGGAGACGGTGACCTGGGT-3' (SEQ ID NO: 140); and Primer Rev-2: 5'- GATGTGCAGCTGCAGGAGTCTGGRGGAGG-3' (SEQ ID NO: 141).

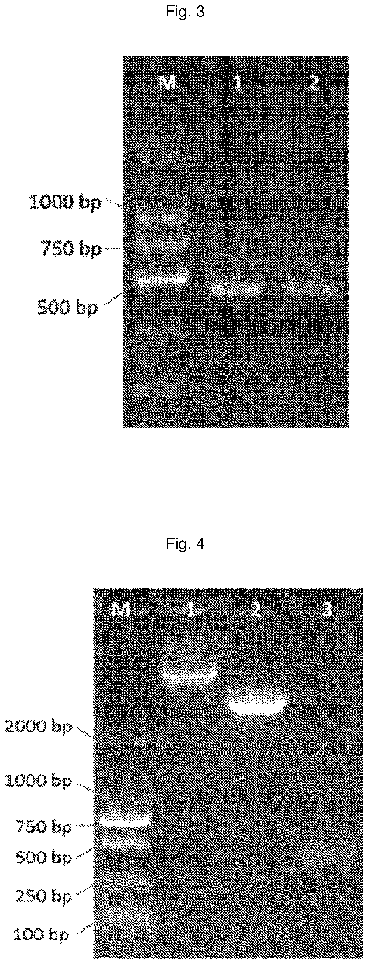

The obtained PCR product performs the agarose gel electrophoresis, an agarose gel electrophoresis image was shown in Fig. 3, the DNA product gel recovery kit was used to recover, and the ultraviolet spectrophotometer was used to quantify. Finally, 200 µL of the target gene (VHH) of about 500 bp was obtained, and the concentration was 400 ng/µL.

### 1.1.5 Library transformation

The obtained target gene and vector pHEN1 were double-digested with Sfi I and Not I, an enzyme-digested product performs the agarose gel electrophoresis, and results were shown in Fig. 4. The digested target gene and pHEN1 were linked by a T4 DNA ligase, then transformed into an *Escherichia coli* electro-transformation competent cell TG1, and a single domain antibody gene library against the SARS-COV-2-Spike protein was constructed, which was named J2-Lib. There was a total of 15 transformations, and after mixing uniformly, it was evenly spread on 6 Φ150 mm petri dishes (a Luria-Bertani (LB) solid medium containing ampicillin).

0.1 µL, 0.01 µL, 0.001 µL and 0.0001 µL of mixed transformation solution were taken and spread evenly on a Φ90mm petri dish (the LB solid medium containing the ampicillin) for the calculation of library capacity (a plate of which the number of colonies was 30 -300 was taken as a quasi count), as shown in Table 5, the calculated library capacity was 1.395×10⁸ cfu.

**Table 5**

| | | | | |
|---|---|---|---|---|
| Bacterial solution volume (µL) | 0.1 | 0.01 | 0.001 | 0.0001 |
| Colony count | Full plate | 93 | 12 | 1 |

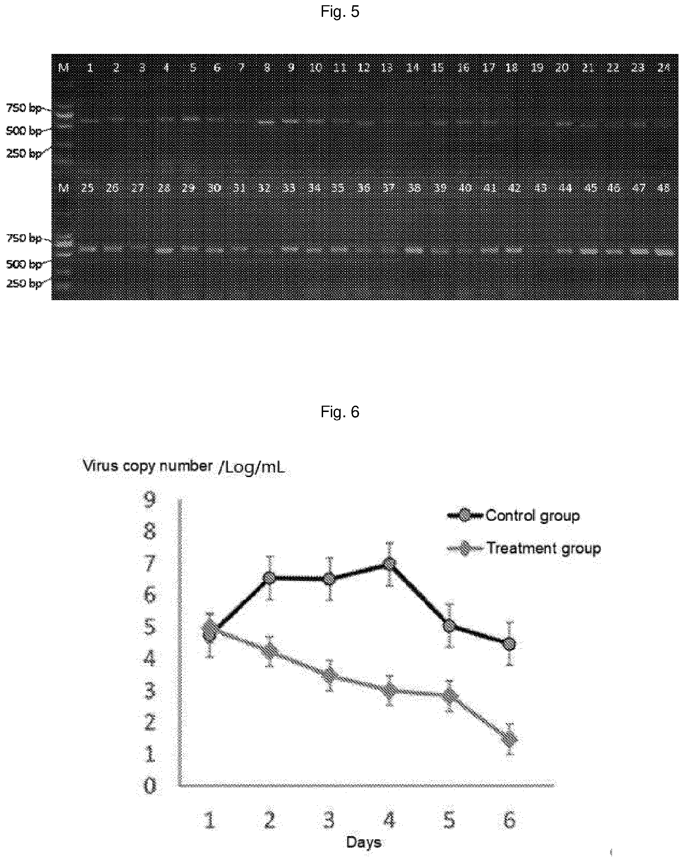

48 colonies were randomly selected in the above petri dish for calculating the library capacity, the colony PCR was performed, and the PCR products perform the agarose gel electrophoresis to calculate the target gene insertion rate of the library. The agarose gel electrophoresis was shown in Fig. 5, it was indicated that the insertion rate of the library was 100%, and the actual library capacity of the library was 1.395×10⁸ cfu.

A colony PCR system was as follows: (Table 6)

**Table 6**

| | |
|---|---|
| 10×Ex Taq Buffer (Mg²⁺ plus) | 5 µL |
| dNTP mixture(each 2.5 mM) | 4 µL |
| Primer For-1 (10 mM each) | 1.5 µL |
| Primer Rev-1 (10 mM each) | 1.5 µL |
| Colony suspension | 1 µL |
| Ex Taq enzyme | 0.25 µL |
| H₂O | Up to 50 µL |

The colony PCR reaction conditions were: 98°C for 10 s, 50°C for 30 s, 72°C for 1 min, and a total of 30 cycles.

### 1.1.6 Library rescue

10-100 times of the library capacity of live cells were taken from the above J2-Lib gene library for inoculation and culture. After being cultured to a log phase, an M13K07 phage was used for rescue. After rescue culture, the phage was collected by centrifugation, and the phage was purified by PEG-NaCl, namely a phage display library was obtained, and named J2-PDL, with a titer of 3.4×10¹³ cfu/mL. It may be directly used for subsequent affinity screening of the specific phages.

### 1.2 Screening of single domain antibodies against SARS-COV-2-Spike protein

A plate was coated with 3 µg/well of the Spike-RBD protein (spike protein receptor binding domain protein), and placed at 4°C overnight; it was blocked with 1wt% nonfat dry milk at a room temperature for 2 h, 100 µl of a phage (8×10¹¹ tfu, from the phage display library J2-PDL constructed in 1.1.6) was added, and acted at the room temperature for 1 h. After that, it was eluted with PBST (phosphate buffer solution (PBS) containing 0.05vt% Tween 20) for 5 times, and the unbound phage was washed away; the phage specifically bound to the Spike-RBD protein was dissociated with triethylamine (100 mM), *Escherichia coli* TG1 in the log phase growth was infected, and the phage was generated and purified for the next round of screening. The same screening process was repeated for 3-4 rounds. Thus, positive clones were enriched, and the purpose of screening Spike-RBD protein-specific antibodies in the antibody library with the phage display library was achieved. The obtained positive phages were sequenced, to obtain the antibody gene sequences.

The obtained antibody gene sequences were constructed on a pcDNA3.4 vector respectively, the antibody was expressed in a HEK-293 cell, and the antibody in a supernatant of the culture medium was collected by purification with a protein A medium. The purified antibody was incubated with the Spike-RBD-coated plate for an enzyme-linked immunosorbent assay (ELISA). The antibody that may specifically bind to the Spike-RBD protein was obtained.

The obtained antibody sequences were analyzed according to sequence alignment software Vector NTI. The clones with the same CDR1, CDR2 and CDR3 sequences were regarded as the same antibody strain, and the clones with the different CDR sequences were regarded as the different antibody strains. A total of 27 different single domain antibody strains were obtained, and the single domain antibody sequences, such as SEQ ID NOs: 82-108, respectively carry 27 groups of CDR1-3 sequences in SEQ ID NOs: 1-81, as shown specifically in Table 7.

**Table 7**

| Single domain antibody | CDR1 | CDR2 | CDR3 |
|---|---|---|---|
| COV2-114-1 | SEQ ID NO:1 | SEQ ID NO:2 | SEQ ID NO:3 |
| COV2-187-2 | SEQ ID NO:4 | SEQ ID NO:5 | SEQ ID NO:6 |
| COV2-360-3 | SEQ ID NO:7 | SEQ ID NO:8 | SEQ ID NO:9 |
| COV2-143-4 | SEQ ID NO:10 | SEQ ID NO:11 | SEQ ID NO:12 |
| COV2-13-5 | SEQ ID NO:13 | SEQ ID NO:14 | SEQ ID NO:15 |
| COV2-356-6 | SEQ ID NO:16 | SEQ ID NO:17 | SEQ ID NO:18 |
| COV2-115-7 | SEQ ID NO:19 | SEQ ID NO:20 | SEQ ID NO:21 |
| COV2-22-8 | SEQ ID NO:22 | SEQ ID NO:23 | SEQ ID NO:24 |
| COV2-116-9 | SEQ ID NO:25 | SEQ ID NO:26 | SEQ ID NO:27 |
| COV2-122-10 | SEQ ID NO:28 | SEQ ID NO:29 | SEQ ID NO:30 |
| COV2-171-11 | SEQ ID NO:31 | SEQ ID NO:32 | SEQ ID NO:33 |
| COV2-136-12 | SEQ ID NO:34 | SEQ ID NO:35 | SEQ ID NO:36 |
| COV2-237-13 | SEQ ID NO:37 | SEQ ID NO:38 | SEQ ID NO:39 |
| COV2-353-14 | SEQ ID NO:40 | SEQ ID NO:41 | SEQ ID NO:42 |
| COV2-121-15 | SEQ ID NO:43 | SEQ ID NO:44 | SEQ ID NO:45 |
| COV2-324-16 | SEQ ID NO:46 | SEQ ID NO:47 | SEQ ID NO:48 |
| COV2-120-17 | SEQ ID NO:49 | SEQ ID NO:50 | SEQ ID NO:51 |
| COV2-153-18 | SEQ ID NO:52 | SEQ ID NO:53 | SEQ ID NO:54 |
| COV2-39-19 | SEQ ID NO:55 | SEQ ID NO:56 | SEQ ID NO:57 |
| COV2-124-20 | SEQ ID NO:58 | SEQ ID NO:59 | SEQ ID NO:60 |
| COV2-18-21 | SEQ ID NO:61 | SEQ ID NO:62 | SEQ ID NO:63 |
| COV2-119-22 | SEQ ID NO:64 | SEQ ID NO:65 | SEQ ID NO:66 |
| COV2-151-23 | SEQ ID NO:67 | SEQ ID NO:68 | SEQ ID NO:69 |
| COV2-214-24 | SEQ ID NO:70 | SEQ ID NO:71 | SEQ ID NO:72 |
| COV2-331-25 | SEQ ID NO:73 | SEQ ID NO:74 | SEQ ID NO:75 |
| COV2-32-26 | SEQ ID NO:76 | SEQ ID NO:77 | SEQ ID NO:78 |
| COV2-336-27 | SEQ ID NO:79 | SEQ ID NO:80 | SEQ ID NO:81 |

### 1.3 Evaluation and identification of single domain antibody against SARS COV-2-Spike protein

### 1.3.1 Expression and purification of single domain antibody in host Escherichia coli

The obtained gene coding sequences of 27 single domain antibodies of different CDR1-3 were recombined to an expression vector PET32b (Novagen, product number: 69016-3), and recombinant plasmids with correct sequencing identification were respectively transformed to an expression host strain BL1 (DE3) (Tiangen Biochemical Technology, product number: CB105-02), it was spread on an LB plate containing 100 µg/mL of ampicillin, and cultured at 37°C overnight. A single colony was selected to inoculate, and cultured overnight. On the second day, the overnight strain was transferred and amplified. It was cultured in a shaker at 37°C until the OD value reaches 0.5-1, 0.5 mM of isopropyl-β-d-thiogalactoside (IPTG) was added for induction, and it was cultured in the shaker at 28°C overnight. On the second day, strains were collected by centrifugation, and the collected strains were broken to obtain a crude antibody extract. Then, 27 single domain antibody proteins were purified, so that its purity was above 90%.

### 1.3.2 Competitive ELISA to investigate blocking effect of SARS-COV-2-Spike protein single domain antibody on binding of Spike-RBD protein to receptor ACE2

Firstly, Spike-RBD protein and ACE2 protein were obtained by expressing HEK293 cells (pCDNA4, Invitrogen, Cat V86220). Then, a Biotinlytion kit of Thermo Company was used to, obtain a biotinylated ACE2 protein.

0.5 µg/well of the Spike-RBD protein was used to coat a plate overnight at 4°C. After that, 100 ng of the single domain antibody purified in 1.3.1 and 5 µg of the biotinylated ACE2 protein were added per well, and a control group was set. The single domain antibody was not added to wells of the control group 1, the biotinylated ACE2 protein was not added to wells of a control group 2, and it was reacted at the room temperature for 2 h. After that, SA-HRP (purchased from Sigma Company) was added, and after reacting at the room temperature for 1 hour, color developing solution was added, and the absorption value was read at the wavelength of 450 nm. While the OD value of a sample was less than 0.8 compared to the OD value of the control, it was considered that the single domain antibody has the blocking effect.

As shown in Table 8, 27 different single domain antibody strains show the blocking effect on Spike protein/ACE2 protein interaction.

**Table 8**

| Sample | OD |
|---|---|
| Control group 1 | 2.250 |
| Control group 2 | 0.041 |
| COV2-114-1 | 0.213 |
| COV2-187-2 | 0.181 |
| COV2-360-3 | 0.132 |
| COV2-143-4 | 0.157 |
| COV2-13-5 | 0.195 |
| COV2-356-6 | 0.192 |
| COV2-115-7 | 0.216 |
| COV2-22-8 | 0.412 |
| COV2-116-9 | 0.388 |
| COV2-122-10 | 0.293 |
| COV2-171-11 | 0.358 |
| COV2-136-12 | 0.196 |
| COV2-237-13 | 0.147 |
| COV2-353-14 | 0.223 |
| COV2-121-15 | 0.361 |
| COV2-324-16 | 0.431 |
| COV2-120-17 | 0.382 |
| COV2-153-18 | 0.153 |
| COV2-39-19 | 0.248 |
| COV2-124-20 | 0.328 |
| COV2-18-21 | 0.384 |
| COV2-119-22 | 0.258 |
| COV2-151-23 | 0.152 |
| COV2-214-24 | 0.423 |
| COV2-331-25 | 0.169 |
| COV2-32-26 | 0.188 |
| COV2-336-27 | 0.197 |

In a laboratory with a biosafety level of P3, 27 strains of the single domain antibodies were added to a culture system respectively by infecting a VERO cell model with viruses, and the specific operation was as follows: 10⁴/well of VERO cells were added to a 96-well plate, and after 24 hours, it was washed twice with PBS, 27 strains of the single domain antibodies were respectively mixed with the viruses and added to the 96-well plate. The initial concentration of the antibody was 100 µg/mL, then it was respectively diluted by 2 times in 10 gradients, 5 wells were duplicated, and it was incubated at 37 degrees for 2 hours. On the 5th day, it was detected whether the VERO cells have pathological changes. If the cells do not have the pathological changes after being treated with the single domain antibody, it was indicated that the single domain antibody has the effect of neutralizing the virus and blocking the virus infection of the VERO cells.

Test results showed that after the treatment with the above 27 strains of the single domain antibodies, the VERO cells do not have the pathological changes, while most of the VERO cells to which only the viruses were added but the single domain antibodies were not added suffer from the pathological changes and die, it was indicated that all the 27 strains of the single domain antibodies obtained may block the process of the viruses infecting the cells, and it was shown that it is an effective neutralizing antibody.

### Embodiment 2

### 1.1 Preparation of Fc fusion protein of SARS-COV-2-Spike protein single domain antibody

According to a constant region amino acid sequence of a human immunoglobulin (lgG1) on the protein database Uniprot, the amino acid sequence (SEQ ID NO: 109) of the human IgG1-Fc region was obtained. A nucleic acid fragment encoding the human IgG1-Fc (the nucleic acid sequence was as shown in SEQ ID NO: 137) was obtained from the total RNA of a human peripheral blood mononuclear cell (PBMC) by a reverse transcription PCR. A nucleic acid fragment encoding a fusion protein of the SARS-COV-2-Spike protein single domain antibody and Fc was obtained by an overlapping PCR, and was recombined to a vector pCDNA4 (Invitrogen, Cat V86220).

A successfully constructed pCDNA4 plasmid containing the nucleic acid fragment of the fusion protein of the SARS-COV-2-Spike protein single domain antibody and Fc was transfected into the HEK293 cell for expression. Specifically, the recombinant expression plasmid was diluted with a Freestyle293 medium and added with polyethylenimine (PEI) solution required for transformation. A plasmid/PEI mixture was respectively added to HEK293 cell suspension and placed in a shaker with 10% CO₂ at 37°C and 100 rpm for culture; and 50 µg/L of IGF-1 was replenished. After 4 h, an EX293 medium, 2 mM of glutamine and 50 µg/L of IGF-1 were added, and shake culture was performed at 120 rpm. After 24 h, 3.8 mM of a valproic acid (VPA) was added. After 5 days of culture, a supernatant of expression culture was collected, and purified by a Protein A affinity chromatography, to obtain the fusion protein of the SARS-COV-2-Spike protein single domain antibody and Fc.

The sequences of the obtained fusion proteins of 27 SARS-COV-2-Spike protein single domain antibodies and Fc were as shown in SEQ ID NOs: 110-136.

### 1.2 Identification of functions of fusion protein of SARS-COV-2-Spike protein single domain antibody and Fc

The binding ability of the fusion protein of SARS-COV-2-Spike protein single domain antibody and Fc to the SARS-COV-2-Spike protein was identified by a surface plasmon resonance (SPR) method. The specific operation was as follows: the binding kinetics of 27 strains of the fusion protein of SARS-COV-2-Spike protein single domain antibody and Fc against spike-RBD was measured by the SRP method with a BIAcoreX100 instrument, and the spike-RBD protein was directly coated on a CM5 biosensor chip to obtain about 1000 response units (RU). For the kinetic measurement, the fusion protein of SARS-COV-2-Spike protein single domain antibody and Fc was continuously diluted (1.62 nm to 1000 nm) by three times with HBS-EP+1 × buffer solution (GE, cat # BR-1006-69), a sample was loaded at 25°C for 120 s, the dissociation time was 30 min, and 10 mM of glycine-HCl (pH 2.0) was added and regenerated for 120 s. A simple one-to-one Languir binding model (BIAcore Evaluation Software version 3.2) was used to calculate the binding rate (kon) of the fusion protein to the SARS-COV-2-Spike protein, the dissociation rate (koff) and the equilibrium dissociation constant (kD) (calculated by the ratio koff/kon). Calculation results were shown in Table 9.

**Table 9**

| Antibody number | Binding rate (kon) | Dissociation rate (koff) | Equilibrium dissociation constant (kD;koff/kon) |
|---|---|---|---|
| COV2-114-1-Fc | 1.74E+06 | 1.52E-05 | 0.87E-12 |
| COV2-187-2-Fc | 1.52E+05 | 3.26E-05 | 2.14E-11 |
| COV2-360-3-Fc | 2.12E+06 | 3.45E-04 | 1.63E-11 |
| COV2-143-4-Fc | 3.12E+06 | 6.25E-05 | 2.00E-12 |
| COV2-13-5-Fc | 1.45E+06 | 1.23E-05 | 0.85E-12 |
| COV2-356-6-Fc | 5.23E+05 | 5.68E-04 | 1.09E-10 |
| COV2-115-7-Fc | 1.25E+05 | 1.68E-05 | 1.34E-11 |
| COV2-22-8-Fc | 1.89E+05 | 5.96E-04 | 3.15E-10 |
| COV2-116-9-Fc | 2.56E+06 | 6.84E-05 | 2.67E-12 |
| COV2-122-10-Fc | 3.51E+06 | 9.23E-05 | 2.63E-12 |
| COV2-171-11-Fc | 4.23E+06 | 4.26E-05 | 1.01E-12 |
| COV2-136-12-Fc | 5.36E+04 | 5.63E-04 | 1.05E-9 |
| COV2-237-13-Fc | 5.47E+06 | 5.78E-05 | 1.06E-12 |
| COV2-353-14-Fc | 9.23E+06 | 6.34E-05 | 0.69E-12 |
| COV2-121-15-Fc | 1.29E+06 | 2.68E-05 | 2.08E-12 |
| COV2-324-16-Fc | 4.85E+06 | 3.71E-05 | 0.76E-12 |
| COV2-120-17-Fc | 6.52E+07 | 3.64E-04 | 0.56E-12 |
| COV2-153-18-Fc | 2.56E+06 | 3.15E-05 | 1.23E-12 |
| COV2-39-19-Fc | 1.89E+05 | 2.46E-05 | 1.30E-11 |
| COV2-124-20-Fc | 3.62E+06 | 3.51E-05 | 0.97E-12 |
| COV2-18-21-Fc | 5.23E+06 | 3.99E-05 | 0.76E-12 |
| COV2-119-22-Fc | 1.86E+06 | 4.22E-05 | 2.27E-12 |
| COV2-151-23-Fc | 2.58E+06 | 3.22E-05 | 1.25E-12 |
| COV2-214-24-Fc | 6.32E+06 | 1.29E-05 | 0.20E-12 |
| COV2-331-25-Fc | 4.23E+06 | 3.56E-05 | 0.84E-12 |
| COV2-32-26-Fc | 4.26E+06 | 3.14E-05 | 0.74E-12 |
| COV2-336-27-Fc | 1.56E+06 | 6.31E-05 | 4.04E-12 |

It may be seen from Table 9 that the binding rate of the fusion protein of SARS-COV-2-Spike protein single domain antibody and Fc to the SARS-COV-2-Spike protein was higher, the dissociation rate was lower, and the equilibrium dissociation constant KD was less than 1.05 E-9, it was indicated that the fusion protein may bind to the SARS-COV-2-Spike protein more quickly and was difficult to dissociate. It was further indicated that as a blocking antibody, the fusion protein of SARS-COV-2-Spike protein single domain antibody and Fc has the excellent blocking effect.

### 1.3 Identification of blocking ability of SARS-COV-2-Spike protein single domain antibody and Fc fusion protein to interaction of Spike protein/ACE2 by competitive ELISA

An ACE2 protein was obtained by expressing the HEK293 cells. A biotinylated protein ACE2-Biotin was obtained by the Biotinlytion kit of Thermo Company.

0.5 µg/well of the Spike-RBD protein was used to coat a plate at 4°C overnight. After that, 200 ng of 27 strains of obtained SARS-COV-2-Spike protein single domain antibody and Fc fusion proteins and 5 µg of ACE2-Biotin were added per well. The fusion protein was not added in the control group 1, ACE2-Biotin was not added in the control group 2, and a reaction was performed at the room temperature for 2 h. After being washed, SA-HRP (purchased from Sigma Company) was added, and reacted at the room temperature for 1 hour. After being washed, the color developing solution was added, and the absorption value was read at 450 nm of the wavelength. Results were shown in Table 10.

**Table 10**

| Sample | OD |
|---|---|
| Control group 1 | 2.319 |
| Control group 2 | 0.021 |
| COV2-114-1 | 0.135 |
| COV2-187-2 | 0.163 |
| COV2-360-3 | 0.175 |
| COV2-143-4 | 0.129 |
| COV2-13-5 | 0.195 |
| COV2-356-6 | 0.137 |
| COV2-115-7 | 0.253 |
| COV2-22-8 | 0.321 |
| COV2-116-9 | 0.297 |
| COV2-122-10 | 0.203 |
| COV2-171 -11 | 0.192 |
| COV2-136-12 | 0.196 |
| COV2-237-13 | 0.107 |
| COV2-353-14 | 0.256 |
| COV2-121-15 | 0.410 |
| COV2-324-16 | 0.302 |
| COV2-120-17 | 0.188 |
| COV2-153-18 | 0.124 |
| COV2-39-19 | 0.199 |
| COV2-124-20 | 0.278 |
| COV2-18-21 | 0.264 |
| COV2-119-22 | 0.156 |
| COV2-151-23 | 0.201 |
| COV2-214-24 | 0.303 |
| COV2-331-25 | 0.142 |
| COV2-32-26 | 0.167 |
| COV2-336-27 | 0.129 |

The results showed that the fusion protein of SARS-COV-2-Spike protein single domain antibody and Fc may effectively block the interaction between the Spike protein and ACE2.

### 1.4 Analysis of binding specificity of SARS-COV-2-Spike protein single domain antibody and Fc fusion protein to Spike protein

The human HEK293 cells were transiently transfected, to obtain plasmids (pCDNA4, Invitrogen, Cat V86220) with 7 currently-known coronaviruses (SARS-COV-2, HCoV-229E, HCoV-OC43, HCoV-NL63, HCoV-HKU1, SARS-CoV, and MERS-CoV) Spike protein full-length genes, and the Spike protein was transiently expressed on a membrane. This plasmid makes a C-terminal of the Spike protein fused with an enhanced green fluorescent protein (EGFP), so that the expression level of the Spike protein on the membrane may be investigated by the green fluorescence intensity. The constructed cells were resuspended in 0.5% of a PBS-BSA Buffer, and the fusion protein of SARS-COV-2-Spike protein single domain antibody and Fc was added. At the same time, a negative control was set, and incubated on ice for 20 min. After being washed, an eBioscience secondary antibody anti-hlg-PE was added, and put on ice for 20 min. After being washed, the cells were resuspended in 500 µl of the 0.5% PBS-BSA Buffer, and detected by a flow cytometer. Results showed that 27 strains of the SARS-COV-2-Spike protein single domain antibody-Fc fusion proteins only specifically bind to the SARS-COV-2-Spike protein, and do not bind to the Spike proteins of other coronaviruses.

### 1.5 Blocking of SARS-COV-2 to infect cells by SARS-COV-2-Spike protein single domain antibody and Fc fusion protein

15 healthy rhesus monkeys with early administration (100 µg of the fusion protein of SARS-COV-2-Spike protein single domain antibody and Fc/rhesus monkey) and 15 healthy rhesus monkeys without the early administration were fed in the same environment for 10 days with 2 rhesus monkeys infected with SARS-COV-2. After 10 days, it was found from observation that the 15 healthy rhesus monkeys with the early administration have no abnormality, and viral nucleic acid tests were all negative; however, 12 of the 15 healthy rhesus monkeys without the early administration showed symptoms of infection, and they were all positive by the viral nucleic acid tests.

The above test results showed that the SARS-COV-2-Spike protein single domain antibody provided by the present application may completely block the infection of SARS-COV-2 and has the excellent preventive effect.

6 of the above 12 rhesus monkeys infected with SARS-COV-2 and having the symptoms (treatment group) were treated by administration of the fusion protein of SARS-COV-2-Spike protein single domain antibody and Fc (100 µg of the fusion protein of SARS-COV-2-Spike protein single domain antibody and Fc/rhesus monkey) provided by the present application, and the other 6 rhesus monkeys (control group) were not treated by administration. Within 6 days after the treatment, the viral load was detected once a day. After being detected, the average load of SARS-COV-2 per milliliter of blood in 6 rhesus monkeys in the treatment group was significantly lower than that in the control group, as shown in Fig. 6. Herein the detection process of the load of SARS-COV-2 was as follows: the blood of the rhesus monkeys (treatment group) treated by the administration and the rhesus monkeys (control group) untreated by the administration was respectively taken, and the nucleic acids of the viruses in the blood were extracted for detection. The detection process was as follows: RNA of SARS-COV-2 was extracted with a RNA extraction kit (Qiagen) according to instructions, and the RNA obtained was dissolved in 50 µL of an elution buffer and used as a template for RT-PCR amplification. A RT-PCR product was used as a template, and primers RBD-qF1 (5'-CAATGGTTTAACAGGCACAGG-3', SEQ ID NO: 142) and RBD-qR1 (5'-CTCAAGTGTCTGTGGATCACG-3', SEQ ID NO: 143) were used to amplify an S region gene of the virus. A HiScriptR II One Step qRT-PCR SYBRRGreen Kit (Vazyme Biotech Co., Ltd) was used, and the operation was performed according to the kit instructions. The PCR amplification conditions were set as follows: 50°C for 3 min, 95°C for 10 s, 60°C for 30 s, and 40 cycles. The PCR amplification instrument used was an ABI quantitative PCR instrument.

The above in vivo experimental results showed that the fusion protein of SARS-COV-2-Spike protein single domain antibody and Fc of the present application has the significant effect of inhibiting SARS-COV-2 to infect the cells and amplifying in the rhesus monkeys infected with SARS-COV-2.

### 1.6 Stability research of SARS-COV-2-Spike protein single domain antibody and Fc fusion protein

500 mM of ammonium bicarbonate was used as an alkaline destruction reagent, and treated at 37°C for 48 h. 1% hydrogen peroxide was selected as an oxidant, and treated at the room temperature for 10 h.

The biological activity changes of the above 27 SARS-COV-2-Spike protein single domain antibody and Fc fusion proteins before and after the treatment were detected by the competitive ELISA. The average competitive ELISA activity after 48 h of the alkali treatment was 107% compared with that after 0 h of the alkali treatment; and the average competitive ELISA activity after 10 h of the oxidation was 111% compared with that after 0 h of the oxidation.

This showed that the 27 SARS-COV-2-Spike protein single domain antibody and Fc fusion proteins provided in the present application have the higher stability.

The above are only preferred embodiments of the present application and are not intended to limit the present application. Any modifications, equivalent replacements or improvements made within the spirit and principle of the present application shall be included in a scope of protection of the present application.

## Claims

1. A SARS-COV-2 spike protein binding molecule, specifically binding to a SARS-COV-2 spike protein and comprising at least one immunoglobulin single variable domain, and CDR1, CDR2 and CDR3 in the immunoglobulin single variable domain are selected from any one of the following combinations:
1) CDR1 shown in SEQ ID NO: 1, CDR2 shown in SEQ ID NO: 2 and CDR3 shown in SEQ ID NO: 3;
2) CDR1 shown in SEQ ID NO: 4, CDR2 shown in SEQ ID NO: 5 and CDR3 shown in SEQ ID NO: 6;
3) CDR1 shown in SEQ ID NO: 7, CDR2 shown in SEQ ID NO: 8 and CDR3 shown in SEQ ID NO: 9;
4) CDR1 shown in SEQ ID NO: 10, CDR2 shown in SEQ ID NO: 11 and CDR3 shown in SEQ ID NO: 12;
5) CDR1 shown in SEQ ID NO: 13, CDR2 shown in SEQ ID NO: 14 and CDR3 shown in SEQ ID NO: 15;
6) CDR1 shown in SEQ ID NO: 16, CDR2 shown in SEQ ID NO: 17 and CDR3 shown in SEQ ID NO: 18;
7) CDR1 shown in SEQ ID NO: 19, CDR2 shown in SEQ ID NO: 20 and CDR3 shown in SEQ ID NO: 21;
8) CDR1 shown in SEQ ID NO: 22, CDR2 shown in SEQ ID NO: 23 and CDR3 shown in SEQ ID NO: 24;
9) CDR1 shown in SEQ ID NO: 25, CDR2 shown in SEQ ID NO: 26 and CDR3 shown in SEQ ID NO: 27;
10) CDR1 shown in SEQ ID NO: 28, CDR2 shown in SEQ ID NO: 29 and CDR3 shown in SEQ ID NO: 30;
11) CDR1 shown in SEQ ID NO: 31, CDR2 shown in SEQ ID NO: 32 and CDR3 shown in SEQ ID NO: 33;
12) CDR1 shown in SEQ ID NO: 34, CDR2 shown in SEQ ID NO: 35 and CDR3 shown in SEQ ID NO: 36;
13) CDR1 shown in SEQ ID NO: 37, CDR2 shown in SEQ ID NO: 38 and CDR3 shown in SEQ ID NO: 39;
14) CDR1 shown in SEQ ID NO: 40, CDR2 shown in SEQ ID NO: 41 and CDR3 shown in SEQ ID NO: 42;
15) CDR1 shown in SEQ ID NO: 43, CDR2 shown in SEQ ID NO: 44 and CDR3 shown in SEQ ID NO: 45;
16) CDR1 shown in SEQ ID NO: 46, CDR2 shown in SEQ ID NO: 47 and CDR3 shown in SEQ ID NO: 48;
17) CDR1 shown in SEQ ID NO: 49, CDR2 shown in SEQ ID NO: 50 and CDR3 shown in SEQ ID NO: 51;
18) CDR1 shown in SEQ ID NO: 52, CDR2 shown in SEQ ID NO: 53 and CDR3 shown in SEQ ID NO: 54;
19) CDR1 shown in SEQ ID NO: 55, CDR2 shown in SEQ ID NO: 56 and CDR3 shown in SEQ ID NO: 57;
20) CDR1 shown in SEQ ID NO: 58, CDR2 shown in SEQ ID NO: 59 and CDR3 shown in SEQ ID NO: 60;
21) CDR1 shown in SEQ ID NO: 61, CDR2 shown in SEQ ID NO: 62 and CDR3 shown in SEQ ID NO: 63;
22) CDR1 shown in SEQ ID NO: 64, CDR2 shown in SEQ ID NO: 65 and CDR3 shown in SEQ ID NO: 66;
23) CDR1 shown in SEQ ID NO: 67, CDR2 shown in SEQ ID NO: 68 and CDR3 shown in SEQ ID NO: 69;
24) CDR1 shown in SEQ ID NO: 70, CDR2 shown in SEQ ID NO: 71 and CDR3 shown in SEQ ID NO: 72;
25) CDR1 shown in SEQ ID NO: 73, CDR2 shown in SEQ ID NO: 74 and CDR3 shown in SEQ ID NO: 75;
26) CDR1 shown in SEQ ID NO: 76, CDR2 shown in SEQ ID NO: 77 and CDR3 shown in SEQ ID NO: 78; and
27) CDR1 shown in SEQ ID NO: 79, CDR2 shown in SEQ ID NO: 80 and CDR3 shown in SEQ ID NO: 81.

2. The SARS-COV-2 spike protein binding molecule as claimed in claim 1, wherein the immunoglobulin single variable domain is a single domain antibody.

3. The SARS-COV-2 spike protein binding molecule as claimed in claim 2, wherein the single domain antibody comprises an amino acid sequence having at least 80% of the sequence identity with any one of SEQ ID NOs: 82-108.

4. The SARS-COV-2 spike protein binding molecule as claimed in claim 2, wherein the single domain antibody comprises an amino acid sequence having at least 90% of the sequence identity with any one of SEQ ID NOs: 82-108.

5. The SARS-COV-2 spike protein binding molecule as claimed in claim 2, wherein the single domain antibody comprises an amino acid sequence having at least 99% of the sequence identity with any one of SEQ ID NOs: 82-108.

6. The SARS-COV-2 spike protein binding molecule as claimed in claim 2, wherein the single domain antibody comprises any one of the amino acid sequences of SEQ ID NOs: 82-108.

7. The SARS-COV-2 spike protein binding molecule as claimed in any one of claims 1-6, wherein the SARS-COV-2 spike protein binding molecule further comprises an immunoglobulin Fc region.

8. The SARS-COV-2 spike protein binding molecule as claimed in claim 7, wherein the immunoglobulin Fc region is a human immunoglobulin Fc region.

9. The SARS-COV-2 spike protein binding molecule as claimed in claim 8, wherein the immunoglobulin Fc region is an Fc region of human lgG1.

10. The SARS-COV-2 spike protein binding molecule as claimed in claim 9, wherein an amino acid sequence of the immunoglobulin Fc region is SEQ ID NO: 109.

11. The SARS-COV-2 spike protein binding molecule as claimed in claim 10, wherein at least one amino acid sequence of SEQ ID NOs: 110-136 is comprised.

12. The SARS-COV-2 spike protein binding molecule as claimed in any one of claims 1-6 and claims 8-11, having at least one of the following characteristics:
a. the KD value of binding to the SARS-COV-2 spike protein is less than 1×10⁻⁸ M;
b. blocking the binding of SARS-COV-2 to a human cell receptor ACE2; and
c. inhibiting the infection and amplification of SARS-COV-2.

13. The SARS-COV-2 spike protein binding molecule as claimed in claim 7, wherein it has at least one of the following characteristics:
a. the KD value of binding to the SARS-COV-2 spike protein is less than 1×10⁻⁸ M;
b. blocking the binding of the SARS-COV-2 spike protein to a human cell receptor ACE2; and
c. inhibiting the infection and amplification of SARS-COV-2.

14. A nucleic acid molecule encoding the SARS-COV-2 spike protein binding molecule as claimed in any one of claims 1-13.

15. An expression vector comprising the nucleic acid molecule as claimed in claim 14 and an expression regulatory element thereof.

16. A host cell comprising and expressing the nucleic acid molecule as claimed in claim 14.

17. A method for acquiring the SARS-COV-2 spike protein binding molecule as claimed in any one of claims 1-13, comprising:
a, culturing the host cell as claimed in claim 16 under a condition that the expression of the SARS-COV-2 spike protein binding molecule is allowed; and
b. collecting the SARS-COV-2 spike protein binding molecule expressed by the host cell from a culture in step a.

18. An immunoconjugate, comprising the SARS-COV-2 spike protein binding molecule as claimed in any one of claims 1-13 conjugated to a therapeutic moiety.

19. A pharmaceutical composition, comprising the SARS-COV-2 spike protein binding molecule as claimed in any one of claims 1-13 and/or the immunoconjugate as claimed in claim 18, and a pharmaceutically acceptable carrier.

20. Use of the pharmaceutical composition as claimed in claim 19 in preparation of a drug for treating or preventing COVID-19.

21. A kit for detecting SARS-COV-2, comprising the SARS-COV-2 spike protein binding molecule as claimed in any one of claims 1-13.

22. A using method of the kit as claimed in claim 21, comprising:
under a condition that the SARS-COV-2 spike protein binding molecule as claimed in any one of claims 1-13 and the SARS-COV-2 spike protein can form a complex,
contacting a detection sample and a control sample with the SARS-COV-2 spike protein binding molecule as claimed in any one of claims 1-13, and detecting the formation of the complex; and
judging the presence of SARS-COV-2 in the sample by a difference in the complex formation between the detection sample and the control sample.
